(19) Europäisches Patentamt
European Patent Office
Office européen des brevets

(11) **EP 2 647 333 A1**

(12) **EUROPEAN PATENT APPLICATION**

(43) Date of publication:
**09.10.2013 Bulletin 2013/41**

(51) Int Cl.:
***A61B 5/00*** *(2006.01)*

(21) Application number: **13157941.9**

(22) Date of filing: **06.03.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **04.04.2012 JP 2012085728**

(71) Applicant: **Canon Kabushiki Kaisha Tokyo 146-8501 (JP)**

(72) Inventor: **Miyasato, Takuro Tokyo, Tokyo 146-8501 (JP)**

(74) Representative: **TBK Bavariaring 4-6 80336 München (DE)**

(54) **Subject information obtaining apparatus and subject information obtaining method**

(57)  A subject information obtaining apparatus includes a plurality of acoustic wave detection elements (e1, e2, e3), each of which detects a photoacoustic wave generated when a subject (30) is irradiated with light and outputs a detection signal, initial-sound-pressure obtaining means (42) that obtains an initial sound pressure in a region of interest (33) in the subject on the basis of the detection signals, light-intensity obtaining means (43) that obtains a corrected light intensity in the region of interest on the basis of weighting coefficients based on sensitivity distributions of the acoustic wave detection elements and a light intensity of the light with which the region of interest is irradiated, and optical-characteristic-value obtaining means (44) that obtains an optical characteristic value in the region of interest on the basis of the initial sound pressure and the corrected light intensity.

## FIG. 1

EP 2 647 333 A1

**Description**

BACKGROUND OF THE INVENTION

Field of the Invention

The present invention relates to a subject information obtaining apparatus and a subject information obtaining method for obtaining subject information by detecting a photoacoustic wave generated when a subject is irradiated with light.

Description of the Related Art

Optical imaging apparatuses which obtain information of an inside of a subject by irradiating the subject with light emitted from a light source, such as a laser, and causing the light to propagate through the subject are intensively studied mainly in the medical field. Photoacoustic imaging (PAI) is one of optical imaging technologies used in such an apparatus. Photoacoustic imaging is a technology for visualizing information regarding an optical characteristic of an inside of a subject (living body) by irradiating the subject with pulsed light emitted from a light source, detecting a photoacoustic wave generated when the light that has propagated and diffused through the subject is absorbed by the subject, and analyzing the detected photoacoustic wave. With this technology, optical characteristic distributions, in particular, an absorption coefficient distribution, an oxygen saturation distribution, etc., in the subject can be obtained.
In photoacoustic imaging, an initial sound pressure $P_0$ of a photoacoustic wave generated from a region of interest of the subject can be expressed as follows:

$$P_0 = \Gamma \cdot \mu_a \cdot \Phi \qquad\qquad \text{Equation (1)}$$

Here, $\Gamma$ is a Gruneisen coefficient, which is calculated by dividing the product of a coefficient of cubical expansion $\beta$ and the square of a sonic speed c by a specific heat at constant pressure $C_P$. It is known that the value of $\Gamma$ is substantially constant when the subject is determined. In addition, $\mu_a$ is an absorption coefficient of the region of interest, and $\Phi$ is a light intensity in the region of interest.
Japanese Patent Laid-Open No. 2010-88627 describes a technology for detecting a variation over time in sound pressure P of a photoacoustic wave that has propagated through a subject with an acoustic wave detector and calculating an initial sound pressure distribution in the subject on the basis of the result of the detection. According to Japanese Patent Laid-Open No. 2010-88627, the product of $\mu_a$ and $\Phi$, that is, an optical energy absorption density, can be obtained by dividing the calculated initial sound pressure by the Gruneisen coefficient $\Gamma$. As is clear from Equation (1), it is necessary to divide the optical energy absorption density by the light intensity $\Phi$ to obtain the absorption coefficient $\mu_a$ from the initial sound pressure Po.

SUMMARY OF THE INVENTION

The present invention in its first aspect provides a subject information obtaining apparatus as specified in claims 1 to 7.
The present invention in its second aspect provides a subject information obtaining method as specified in claims 8 to 10.
The present invention in its third aspect provides a program as specified in claim 11.
Further features of the present invention will become apparent from the following description of exemplary embodiments with reference to the attached drawings.

BRIEF DESCRIPTION OF THE DRAWINGS

Fig. 1 illustrates a subject information obtaining apparatus according to a first embodiment.
Fig. 2 is a flowchart of a subject information obtaining method according to the first embodiment.
Fig. 3 illustrates a subject information obtaining apparatus according to the third embodiment.
Fig. 4 illustrates another subject information obtaining apparatus according to a third embodiment.

DESCRIPTION OF THE EMBODIMENTS

A conversion efficiency with which an acoustic wave detection element converts a photoacoustic wave into a detection signal is dependent on an angle between the normal of a detection surface of the acoustic wave detection element and an incident angle of the acoustic wave. Specifically, the conversion efficiency decreases when the acoustic wave is incident on the detection surface at an angle.

This affects the value of an initial sound pressure obtained through reconstruction based on a detection signal obtained by detecting an acoustic wave with an acoustic wave detector. The thus-obtained initial sound pressure is lower than the actual initial sound pressure.

Accordingly, an optical characteristic value obtained by using the initial sound pressure that is lower than the actual initial sound pressure also differs from the actual value thereof.

Accordingly, an embodiment of the present invention provides a subject information obtaining apparatus and a subject information obtaining method with which an accurate optical characteristic value can be obtained by photoacoustic imaging.

Embodiments of the present invention based on simulations will now be described.

First Embodiment

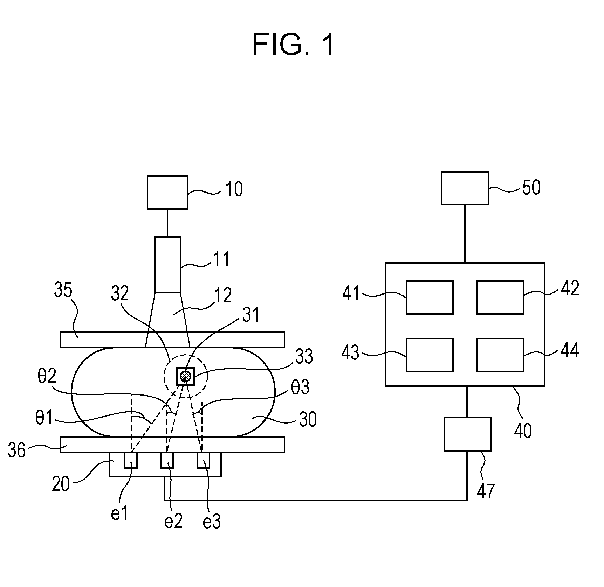

Fig. 1 is a schematic diagram of a subject information obtaining apparatus according to a first embodiment.

In the present embodiment, a subject 30 is secured by being sandwiched between two retaining members 35 and 36. In this state, pulsed light that is emitted from a light source 10 is guided through an optical system 11 and serves as irradiating light 12 with which the subject 30 is irradiated. An acoustic wave detector 20 detects a photoacoustic wave 32 that is generated by a light absorber 31 disposed in the subject 30. The acoustic wave detector 20 includes a first acoustic wave detection element e1, a second acoustic wave detection element e2, and a third acoustic wave detection element e3.

A detection signal obtained by the acoustic wave detector 20 is amplified and converted into a digital signal by a signal collector 47, and is stored in a memory included in a signal processor 40.

The signal processor 40 includes an initial-sound-pressure obtaining module 42, which serves as initial-sound-pressure obtaining means and obtains an initial sound pressure in a region of interest 33 of the subject 30 through image reconstruction by using the detection signal.

The signal processor 40 also includes a light-intensity obtaining module 43, which serves as light-intensity obtaining means and obtains a light intensity in the region of interest 33.

The signal processor 40 also includes an optical-characteristic-value obtaining module 44, which serves as optical-characteristic-value obtaining means and obtains an optical characteristic value in the region of interest 33 by using the initial sound pressure and total light intensity in the region of interest 33.

A display device 50, which serves as a display unit, displays the obtained optical characteristic value.

In the present embodiment, the region of interest refers to a voxel, which is a minimum unit of region that is reconstructed by the initial-sound-pressure obtaining module 42.

The initial-sound-pressure obtaining module 42 is capable of obtaining an initial sound pressure distribution over the entire region of the subject by setting regions of interest over the entire region of the subject 30. Similarly, the light-intensity obtaining module 43 and the optical-characteristic-value obtaining module 44 is capable of respectively obtaining a light intensity distribution and an absorption coefficient distribution over the entire region of the subject by setting regions of interest over the entire region of the subject.

According to the present invention, the light-intensity obtaining module 43 obtains the light intensity on the basis of sensitivity distributions of the acoustic wave detection elements. An accurate absorption coefficient can be obtained by using the light intensity obtained in consideration of the sensitivities of the acoustic wave detection elements.

Example in Which Sensitivities of Acoustic Wave Detection Elements are not Considered

To explain an aspect of the present invention, a simulation example in which the absorption coefficient is obtained without considering the sensitivities of the acoustic wave detection elements will be explained as a comparative example. The comparative example will be explained with reference to the subject information obtaining apparatus illustrated in Fig. 1. In this simulation, the absorption coefficient of the light absorber 31 is set to $\mu_a = 0.088/mm$.

Here, detection signals obtained by the acoustic wave detection elements e1, e2, and e3 illustrated in Fig. 1 and corresponding to the region of interest 33 are defined as $P_{d1}(r_T)$, $Pa_2(r_T)$, and $P_{d3}(r_T)$, respectively.

The light intensities in the region of interest 33 that correspond to the detection signals $P_{d1}(r_T)$, $P_{d2}(r_T)$, and $P_{d3}(r_T)$ are defined as $\Phi_1(r_T)$, $\Phi_2(r_T)$, and $\Phi_3(r_T)$, respectively.

The distance from each acoustic wave detection element to the region of interest 33 is defined as L, the propagation velocity of the photoacoustic wave in the subject is defined as c, and the time at which the subject 30 is irradiated with the irradiating light 12 is defined as t = 0. In this case, the detection signals corresponding to the region of interest are the detection signals obtained by the respective acoustic wave detection elements at the time t = L/c. In addition, the light intensity of the light with which the region of interest 33 is irradiated is the light intensity of the irradiating light 12 in the region of interest 33 at the time t = 0.

In the present embodiment, the region of interest 33 is set at a position $r_T$ where the light absorber 31 is located.

First, the initial- sound- pressure obtaining module 42 obtains an initial sound pressure $P_0$ ($r_T$) in the region of interest 33 by using the detection signals $P_{d1}$ ($r_T$), $P_{d2}$ ($r_T$), and $P_{d3}$ ($r_T$) as expressed in the following Equation (2) .

$$P_0(r_T) \; = \; P_{d1}(r_T) \; + \; P_{d2}(r_T) \; + \; P_{d3}(r_T) \qquad \text{Equation (2)}$$

The detection signals obtained by the acoustic wave detection elements are determined by simulation as follows:

$$P_{d1}(r_T) \; = \; 132 \; Pa$$

$$P_{d2}(r_T) \; = \; 231 \; Pa$$

$$P_{d3}(r_T) \; = \; 198 \; Pa$$

The initial sound pressure is calculated from Equation (2) by using the above parameters as $P_0(r_T)$ = 561 Pa.

Next, the light- intensity obtaining module 43 obtains the light intensity in the subject from, for example, an average optical coefficient of the subject by using a light propagation Monte Carlo method, a transport equation, a light diffusion equation, or the like.

For example, the light-intensity obtaining module 43 calculates light intensities $\Phi_1(r_T)$, $\Phi_2(r_T)$, and $\Phi_3(r_T)$ of the light with which the region of interest 33 is irradiated, the light intensities $\Phi_1(r_T)$, $\Phi_2(r_T)$, and $\Phi_3(r_T)$ corresponding to the detection signals $P_{d1}(r_T)$, $P_{d2}(r_T)$, and $P_{d3}(r_T)$, respectively.

Then, the light- intensity obtaining module 43 obtains an accumulated light intensity $\Phi$ ($r_T$) in the region of interest 33 by adding up the light intensities of the light with which the region of interest 33 is irradiated, the light intensities corresponding to the respective detection signals, as expressed in the following Equation (3) .

$$\Phi(r_T) \; = \; \Phi_1(r_T) \; + \; \Phi_2(r_T) \; + \; \Phi_3(r_T) \qquad \text{Equation (3)}$$

The light intensities of the light with which the region of interest 33 is irradiated, the light intensities corresponding to the respective detection signals, are determined by simulation as follows:

$$\Phi_1(r_T) \; = \; 3750 \; mJ/m^2$$

$$\Phi_2(r_T) \; = \; 3750 \; mJ/m^2$$

$$\Phi_3(r_T) \; = \; 3750 \; mJ/m^2$$

The accumulated light intensity in the region of interest is calculated from Equation (3) by using these parameters as $\Phi(r_T)$ = 11250 mJ/m$^2$.

Next, the optical- characteristic- value obtaining module 44 obtains an absorption coefficient $\mu_a(r_T)$ in the region of interest 33 represented by Equation (4) by using the initial sound pressure $P_0(r_T)$ in the region of interest 33 represented by Equation (2) and the accumulated light intensity $\Phi(r_T)$ in the region of interest 33 represented by Equation (3) . Here, a Gruneisen coefficient $\Gamma$ is $\Gamma$ = 1.

$$\mu_a(r_T) = \frac{P_0(r_T)}{\Phi(r_T)} = \frac{P_{d1}(r_T) + P_{d2}(r_T) + P_{d3}(r_T)}{\Phi_1(r_T) + \Phi_2(r_T) + \Phi_3(r_T)} \qquad \text{Equation (4)}$$

The absorption coefficient in the region of interest 33, which is located at the position $r_T$ of the light absorber, is calculated from Equation (4) by using the above-mentioned parameters as $\mu_a$ = 0.050/mm. On the other hand, the absorption coefficient of the light absorber 31 set in the simulation is $\mu_a$ = 0.088/mm.

Thus, the absorption coefficient determined from Equation (4) is smaller than the set value. This is because the absorption coefficient is calculated without considering the sensitivities of the acoustic wave detection elements.

The present inventor has found that an accurate absorption coefficient can be obtained by weighting the light intensities in consideration of the sensitivities of the acoustic wave detection elements.

Example in Which Sensitivities of Acoustic Wave Detection Elements are Considered

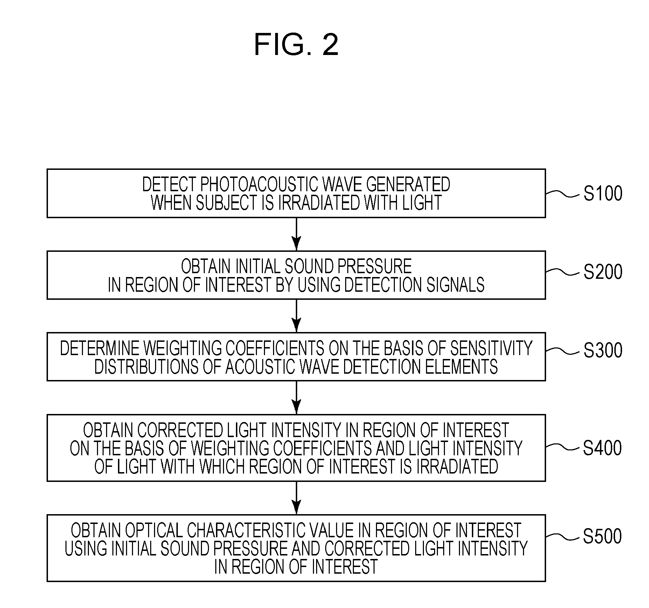

A simulation example according to the present invention in which the absorption coefficient is obtained by using the light intensities in consideration of the sensitivities of the acoustic wave detection elements will now be described with reference to the flowchart illustrated in Fig. 2. The numbers described below are the same as the step numbers illustrated in Fig. 2.

S100: Step of Detecting Photoacoustic Wave Generated When Subject is Irradiated With Light

In this step, the acoustic wave detection elements e1, e2, and e3 detect the photoacoustic wave 32 generated as a result of irradiating the subject 30 with the irradiating light 12.

S200: Step of Obtaining Initial Sound Pressure In Region Of Interest by Using Detection Signals

In this step, the initial-sound-pressure obtaining module 42 obtains the initial sound pressure in the region of interest 33 of the subject 30 by using the detection signals $P_{d1}(r_T)$, $P_{d2}(r_T)$, and $P_{d3}(r_T)$ obtained by the acoustic wave detection elements e1, e2, and e3, respectively, and corresponding to the region of interest 33.

In this step, similar to the method of obtaining the initial sound pressure according to the comparative example, the initial sound pressure is obtained from Equation (2). Therefore, the initial sound pressure obtained by simulation is $P_0(r_T)$ = 561.

S300: Step of Determining Weighting Coefficients on the Basis of Sensitivity Distributions of Acoustic Wave

Detection Elements

In this step, a setting module 41, which is included in the signal processor 40 and serves as a setting unit, sets weighting coefficients on the basis of sensitivity distributions of the acoustic wave detection elements e1, e2, and e3.

In the present embodiment, conversion efficiencies of the acoustic wave detection elements will be explained as the sensitivities of the acoustic wave detection elements.

For example, in the present embodiment, when a photoacoustic wave is incident on an acoustic wave detection element from the front at an angle $\theta$ with respect to the normal of the detection surface of the acoustic wave detection element, a conversion efficiency with which the photoacoustic wave is converted into a detection signal is defined as A($\theta$). Thus, the conversion efficiency is determined by, for example, an angle between a straight line that passes through the region of interest 33 and the detection surface of the acoustic wave detection element and the normal of the detection surface of the acoustic wave detection element, that is, by an incident angle at which the photoacoustic wave generated from the region of interest is incident on the acoustic wave detection element.

When the acoustic wave detection elements e1, e2, and e3 are at angles of $\theta1$, $\theta2$, and $\theta3$, respectively, with respect to the region of interest 33, the conversion efficiencies of the acoustic wave detection elements e1, e2, and e3 based on directivities thereof can be expressed as A($\theta1$), A($\theta2$), and A($\theta3$), respectively.

The conversion efficiencies set in the simulation are as follows:

$$A(\theta 1) \; = \; 0.4$$

$$A(\theta 2) \; = \; 0.7$$

$$A(\theta 3) \; = \; 0.6$$

In an embodiment of the present invention, the sensitivity of each acoustic wave detection element is not limited as long as the sensitivity is based on information lost in a period from the generation of the photoacoustic wave to the conversion thereof into the detection signal. For example, the sensitivity of each acoustic wave detection element may be determined from an attenuation factor by which the photoacoustic wave is attenuated due to diffusion and scattering while the photoacoustic wave travels from the region of interest to the acoustic wave detection element. The attenuation factor may be determined from, for example, the distance between the region of interest and the acoustic wave detection element.

In this step, sensitivities of the acoustic wave detection elements that have been measured in advance may be used. In such a case, table data including the sensitivities of the acoustic wave detection elements in each region of interest may be stored in a memory included in the signal processor 40.

The setting module 41 sets the conversion efficiencies $A(\theta 1)$, $A(\theta 2)$, and $A(\theta 3)$, which serve as the sensitivities of the acoustic wave detection elements e1, e2, and e3, respectively, as the weighting coefficients.

In the present embodiment, the conversion efficiency $A(\theta 1)$ is set as a first weighting coefficient, the conversion efficiency $A(\theta 2)$ is set as a second weighting coefficient, and the conversion efficiency $A(\theta 3)$ is set as a third weighting coefficient. To compensate for the information lost from the generated photoacoustic wave, the sensitivity distribution of each acoustic wave detection element or the product of the sensitivity distribution and a coefficient distribution may instead be used as the weighting coefficient of the acoustic wave detection element.

S400: Step of Obtaining Corrected Light Intensity in Region of Interest on the Basis of Weighting Coefficients and Light Intensity of Light with Which Region of Interest is Irradiated

In this step, the light-intensity obtaining module 43 obtains a corrected light intensity $\Phi'(r_T)$ in the region of interest 33 on the basis of the weighting coefficients $A(\theta 1)$, $A(\theta 2)$, and $A(\theta 3)$ corresponding to the acoustic wave detection elements e1, e2, and e3, respectively, set in S300 and the light intensities $\Phi_1(r_T)$, $\Phi_2(r_T)$, and $\Phi_3(r_T)$ in the region of interest 33 corresponding to the acoustic wave detection elements e1, e2, and e3, respectively.

For example, first, the light-intensity obtaining module 43 obtains a first corrected light intensity in the region of interest 33 on the basis of the weighting coefficient $A(\theta 1)$ based on the sensitivity of the acoustic wave detection element e1 and the light intensity $\Phi_1(r_T)$ of the light with which the region of interest 33 is irradiated. Similarly, a second corrected light intensity and a third corrected light intensity are obtained for the acoustic wave detection elements e2 and e3, respectively, on the basis of the corresponding weighting coefficients and light intensities of the light with which the region of interest 33 is irradiated.

Here, the first to third corrected light intensities are obtained by multiplying the weighting coefficients by the light intensities of the light with which the region of interest is irradiated.

Then, the light-intensity obtaining module obtains the corrected light intensity $\Phi'(r_T)$ in the region of interest 33 by using the first to third corrected light intensities as expressed in the following Equation (5).

$$\Phi'(r_T) \; = \; A(\theta 1) \cdot \Phi_1(r_T) \; + \; A(\theta 2) \cdot \Phi_2(r_T) \; + \; A(\theta 3) \cdot \Phi_3(r_T)$$

$$\text{Equation (5)}$$

As described above, the parameters of Equation (5) are as follows:

$$\Phi_1(r_T) \; = \; 3750 \; mJ/m^2$$

$$\Phi_2(r_T) = 3750 \text{ mJ/m}^2$$

$$\Phi_3(r_T) = 3750 \text{ mJ/m}^2$$

$$A(\theta 1) = 0.4$$

$$A(\theta 2) = 0.7$$

$$A(\theta 3) = 0.6$$

The corrected light intensity in the region of interest 33 can be calculated from Equation (5) by using these parameters as $\Phi'(r_T)$ = 6375 mJ/m$^2$.

The light-intensity obtaining module 43 may  instead obtain the corrected light intensity $\Phi'(r_T)$ by multiplying the product of a light intensity of the light with which the region of interest is irradiated and the number of acoustic wave detection elements by the sum of the sensitivities of all of the acoustic wave detection elements, as expressed in the following Equation (6).

$$\Phi'(r_T) = 3\Phi_1(r_T) \cdot \{A(\theta 1) + A(\theta 2) + A(\theta 3)\} \quad \text{Equation} \quad (6)$$

S500: Step of Obtaining Optical Characteristic Value in Region of Interest on the Basis of Initial Sound Pressure and Corrected Light Intensity in Region of Interest

In this step, the optical-characteristic-value obtaining module 44 obtains an optical characteristic value in the region of interest 33 by using the initial sound pressure $P_0(r_T)$ in the region of interest 33 obtained in S200 and the corrected light intensity $\Phi'(r_T)$ in the region of interest 33 obtained in S400.

For example, the optical-characteristic-value obtaining module 44 obtains the absorption coefficient $\mu_a(r_T)$ in the region of interest 33 represented by the following Equation (7) as an optical characteristic value.

$$\mu_a(r_T) = \frac{P_0(r_T)}{\Phi'(r_T)}$$

$$= \frac{P_{d1}(r_T) + P_{d2}(r_T) + P_{d3}(r_T)}{A(\theta 1) \cdot \Phi_1(r_T) + A(\theta 2) \cdot \Phi_2(r_T) + A(\theta 3) \cdot \Phi_3(r_T)} \quad \text{Equation (7)}$$

The absorption coefficient in the region of interest 33 obtained from Equation (7) by using the above-described parameters is $\mu_a(r_T)$ = 0.088/mm. The absorption coefficient of the light absorber 31 set in the simulation is 0.088/mm. It is clear that the absorption coefficient obtained from Equation (7), which is derived in consideration of the sensitivities of the acoustic wave detection elements, is more accurate than the absorption coefficient obtained from Equation (4), which is derived without considering the sensitivities of the acoustic wave detection elements.

An accurate absorption coefficient of the inside of the subject can be obtained by the above-described steps.

A program including the above-described steps may be executed by the signal processor 40, which serves as a computer.

Although the acoustic wave detector including three acoustic wave detection elements is described in the present embodiment, the present invention may also be applied to a case in which the number of acoustic wave detection elements included in the acoustic wave detector is one, two, or four or more.

Second Embodiment

A subject information obtaining method according to a second embodiment will now be described with reference to the subject information obtaining apparatus illustrated in Fig. 1.

The present embodiment differs from the first embodiment in that the subject is irradiated with multiple lights at different times, and an optical characteristic value is obtained by using photoacoustic waves generated by the respective lights.

First, the optical system 11 guides the pulsed light emitted from the light source 10 so as to irradiate the subject 30 with the irradiating light 12 that serves as first light. A first photoacoustic wave is generated by the light absorber 31 disposed in the subject 30 in response to the irradiation with the first light. The first acoustic wave detection element e1 detects the first photoacoustic wave, so that a first detection signal is obtained.

The subject 30 is also irradiated with the irradiating light 12 that serves as second light at a time different from the time at which the subject 30 is irradiated with the first light. A second photoacoustic wave is generated by the light absorber 31 disposed in the subject 30 in response to the irradiation with the second light. The first acoustic wave detection element e1 detects the second photoacoustic wave, so that a second detection signal is obtained.

Next, the initial-sound-pressure obtaining module 42 obtains the initial sound pressure in the region of interest 33 by using the first detection signal and the second detection signal.

Next, the light-intensity obtaining module 43 obtains the corrected light intensity in the region of interest 33 on the basis of the sensitivity distribution of the first acoustic wave detection element e1, the light intensity of the first light with which the region of interest 33 has been irradiated, and the light intensity of the second light with which the region of interest 33 has been irradiated.

For example, the light-intensity obtaining module 43 obtains a first corrected light intensity in the region of interest on the basis of the sensitivity of the first acoustic wave detection element e1 in the region of interest and the light intensity of the first light with which the region of interest 33 has been irradiated. Similarly, the light-intensity obtaining module 43 obtains a second corrected light intensity in the region of interest on the basis of the sensitivity distribution of the first acoustic wave detection element e1 and the light intensity of the second light with which the region of interest 33 has been irradiated. Then, the light-intensity obtaining module 43 obtains a corrected light intensity in the region of interest 33 by using the first corrected light intensity and the second corrected light intensity.

Next, the optical-characteristic-value obtaining module 44 obtains an optical characteristic value on the basis of the initial sound pressure in the region of interest 33 and the corrected light intensity.

Thus, the optical characteristic value is obtained by using the corrected light intensity obtained on the basis of the light intensities in the region of interest of the first light and the second light, with which the subject is irradiated at different times, and a weighting coefficient based on the sensitivity distribution of an acoustic wave detection element. Accordingly, similar to the first embodiment, an accurate optical characteristic value can be obtained.

According to an embodiment of the present invention, the irradiations with the first light and the second light may either be performed under different irradiation conditions or the same irradiation condition as long as they are performed at different times.

Although the photoacoustic waves are detected by a single acoustic wave detection element according to the present embodiment, embodiments of the present invention may also be applied to a case in which the photoacoustic waves generated by lights emitted at different times are detected by a plurality of acoustic wave detection elements.

A program including the above-described steps may be executed by the signal processor 40, which serves as a computer.

Third Embodiment

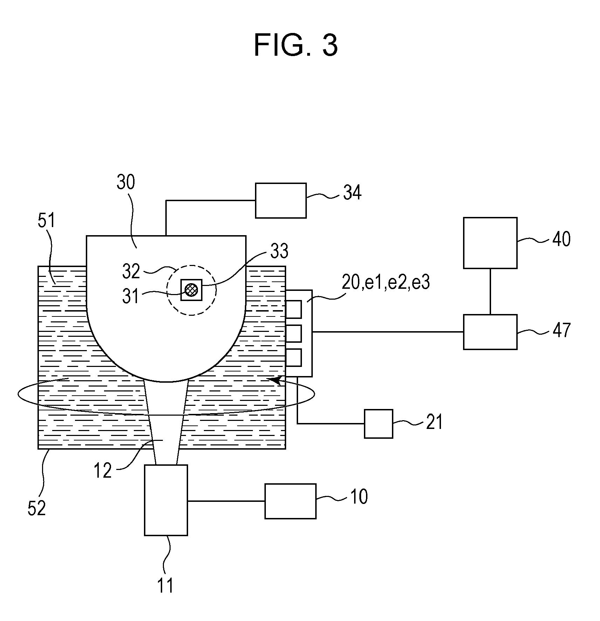

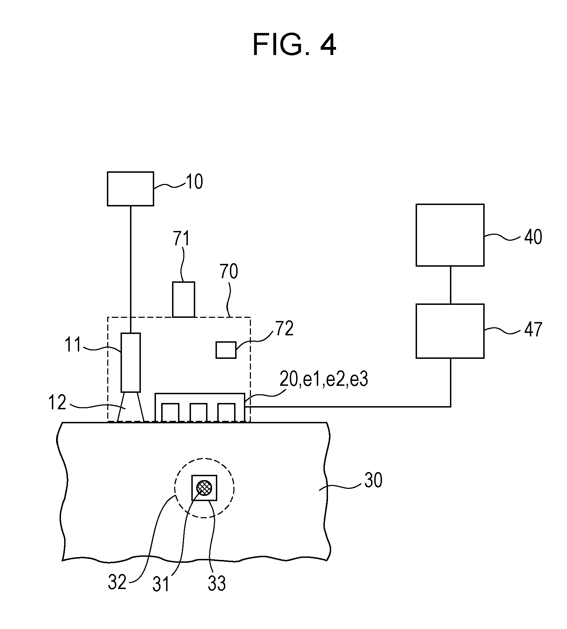

An embodiment of the present invention may also be applied to a subject information obtaining apparatus illustrated in Fig. 3 and a subject information obtaining apparatus illustrated in Fig. 4.

In the subject information obtaining apparatus illustrated in Fig. 3, an acoustic wave detector 20 is rotated around a subject 30 by a detector moving mechanism 21. For example, the detector moving mechanism 21 rotates the acoustic wave detector 20 in the direction shown by the arrow in Fig. 3. A subject moving mechanism 34 is also provided which moves the subject 30 in the up-down, left-right, and front-rear directions with respect to the plane of Fig. 3.

To provide acoustic impedance matching between the subject 30 and the acoustic wave detector 20, the subject 30 is immersed in water 51 which fills a water tank 52. Since the acoustic wave detector 20 rotates around the subject 30, the water tank 52 according to the present embodiment has a columnar shape. The water tank 52 may be formed of, for example, an acrylic that is transparent to irradiating light 12.

The water tank 52 may have, for example, a hemispherical shape instead of a columnar shape as long as the photoacoustic wave can be detected by the acoustic wave detector while the acoustic wave detector is oriented in various directions. Alternatively, the photoacoustic wave can be detected by a plurality of acoustic wave detectors that are oriented in various directions.

With the above-described structure, portions whose shapes cannot be retained by retaining members or the like can also be measured. In addition, since the detection elements may be arranged in many directions with respect to the subject, data having a large amount of information can be obtained.

In the subject information obtaining apparatus illustrated in Fig. 4, an acoustic wave detector 20 and an optical system 11 are disposed in a single housing 70. The housing 70 includes a gripper 71 so that an operator can hold the gripper 71 and move the housing 70. In the example illustrated in Fig. 4, an operator holds the gripper 71 and moves the housing 70 rightward along the plane of Fig. 4 to cause the acoustic wave detection elements to detect the photoacoustic wave.

Unlike the other embodiment, in the present embodiment, the operator holds the gripper 71 and manually moves housing 70 instead of mechanically moving the acoustic wave detector 20. Therefore, the positional relationship between the acoustic wave detector 20 and the region of interest 33 at the time when a photoacoustic wave 32 is detected cannot be determined. However, the positional relationship between the acoustic wave detector 20 and the region of interest 33 needs to be determined to obtain a detection signal corresponding to the region of interest from the detection signal obtained by the acoustic wave detector 20. Therefore, in the present embodiment, the housing 70 may include a position detector 72 that detects the position of the housing 70, that is, the positions of the acoustic wave detector 20 and the optical system 11 contained in the housing 70.

Also in the subject information obtaining apparatuses illustrated in Figs. 3 and 4, an accurate optical characteristic value in the region of interest 33 can be obtained by performing the subject information obtaining method illustrated in Fig. 2.

Other Embodiments

Embodiments of the present invention can also be realized by a computer of a system or apparatus that reads out and executes computer executable instructions recorded on a storage medium (e.g., non-transitory computer-readable storage medium) to perform the functions of one or more of the above-described embodiment(s) of the present invention, and by a method performed by the computer of the system or apparatus by, for example, reading out and executing the computer executable instructions from the storage medium to perform the functions of one or more of the above-described embodiment(s). The computer may comprise one or more of a central processing unit (CPU), micro processing unit (MPU), or other circuitry, and may include a network of separate computers or separate computer processors. The computer executable instructions may be provided to the computer, for example, from a network or the storage medium. The storage medium may include, for example, one or more of a hard disk, a random-access memory (RAM), a read only memory (ROM), a storage of distributed computing systems, an optical disk (such as a compact disc (CD), digital versatile disc (DVD), or Blu-ray Disc (BD)™), a flash memory device, a memory card, and the like.

Structures of main components will now be described.

Light Source 10

The light source 10 is capable of emitting pulsed light of 5 to 50 nanoseconds. Although a high power laser may be used as the light source, a light emitting diode may be used instead of the laser. Various lasers, such as a solid-state laser, a gas laser, a dye laser, and a semiconductor laser, may be used as the laser. Ideally, a Ti:Sa laser pumped by a Nd:YAG laser or an alexandrite laser, which are a high power laser having a continuously variable wavelength, is used. A plurality of single-wavelength lasers having different wavelengths may also be used.

Optical System 11

The pulsed light emitted from the light source 10 is typically guided to the subject while being shaped into a desired optical distribution by optical components, such as a lens and a mirror. However, the pulsed light may instead be propagated through an optical waveguide such as an optical fiber or the like.

The optical system 11 includes, for example, a mirror that reflects light, a lens that collects, magnifies, or changes the shape of light, and a diffusing plate that diffuses light. These optical components are not limited as long as the pulsed light emitted from the light source can be formed into a desired shape before the subject is irradiated therewith. The light can be spread over a certain area instead of being collected by a lens. In such a case, the safety of the subject and the diagnostic region can be increased.

An optical-system moving mechanism for moving the optical system 11 may be provided so that the subject can be scanned with the irradiating light. The optical system may include a plurality of light emitting units so that the irradiating light can be emitted from a plurality of positions.

Acoustic Wave Detector 20

The acoustic wave detector 20 is a detector for detecting a photoacoustic wave generated at a surface and an inside of a subject when the subject is irradiated with light. The acoustic wave detector 20 detects the acoustic wave and converts the acoustic wave into an analog electric signal. The acoustic wave detector 20 may hereinafter be referred to simply as a probe or a transducer. Any type of acoustic wave detector, such as a transducer using a piezoelectric phenomenon, a transducer using optical resonance, or a transducer using a change in capacitance, may be used as long as acoustic wave signals can be detected.

The acoustic wave detector 20 may include a plurality of acoustic wave detection elements that are one-dimensionally or two-dimensionally arranged in an array. When the acoustic wave detection elements that are multi-dimensionally arranged are used, the acoustic wave can be detected simultaneously at a plurality of positions. Therefore, the detection time and the influence of, for example, vibration of the subject can be reduced.

The acoustic wave detector 20 may be configured to be mechanically movable by a detector moving mechanism.

The acoustic wave detector 20 may include a gripper so that an operator can hold the gripper and manually move the acoustic wave detector 20.

Signal Collector 47

The signal collector 47 may be provided which amplifies the electric signal obtained by the acoustic wave detector 20 and converts the electric signal, which is an analog signal, into a digital signal. The signal collector 47 typically includes an amplifier, an A/D converter, and a field programmable gate array (FPGA) chip. In the case where a plurality of detection signals are obtained by the acoustic wave detector, the signal collector 47 may be configured to simultaneously process the plurality of detection signals. In such a case, the time required to form an image can be reduced. In this specification, the concept of "detection signal" includes both the analog signal output from the acoustic wave detector 20 and the digital signal into which the analog signal is converted by the signal collector 47.

Signal Processor 40

The signal processor 40 obtains the optical characteristic value of the inside of the subject by performing, for example, image reconstruction. The signal processor 40 typically includes a workstation, and an image reconstruction process, for example, is performed by software that is programmed in advance. The software used in the workstation includes, for example, the setting module 41, the initial-sound-pressure obtaining module 42, the light-intensity obtaining module 43, and the optical-characteristic-value obtaining module 44.

The modules included in the signal processor 40 may instead be provided as individual devices.

In the case where the modules are formed as hardware, each module may be, for example, an FPGA or an ASIC. Alternatively, each module may be formed as a program for causing the computer to execute the corresponding process.

The signal collector 47 and the signal processor 40 may be integrated with each other. In this case, an optical characteristic value of the subject may be generated by a hardware process instead of a software process performed by a workstation.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions. A subject information obtaining apparatus includes a plurality of acoustic wave detection elements (e1, e2, e3), each of which detects a photoacoustic wave generated when a subject (30) is irradiated with light and outputs a detection signal, initial-sound-pressure obtaining means (42) that obtains an initial sound pressure in a region of interest (33) in the subject on the basis of the detection signals, light-intensity obtaining means (43) that obtains a corrected light intensity in the region of interest on the basis of weighting coefficients based on sensitivity distributions of the acoustic wave detection elements and a light intensity of the light with which the region of interest is irradiated, and optical-characteristic-value obtaining means (44) that obtains an optical characteristic value in the region of interest on the basis of the initial sound pressure and the corrected light intensity.

**Claims**

1.  A subject information obtaining apparatus comprising:

    a plurality of acoustic wave detection elements (e1, e2, e3), each of which detects a photoacoustic wave generated when a subject (30) is irradiated with light and outputs a detection signal;
    initial-sound-pressure obtaining means (42) that obtains an initial sound pressure in a region of interest (33) in the subject (30) on the basis of the detection signals;
    light-intensity obtaining means (43) that obtains a corrected light intensity in the region of interest (33) on the basis of weighting coefficients based on sensitivity distributions of the acoustic wave detection elements (e1, e2, e3) and a light intensity of the light with which the region of interest (33) is irradiated; and
    optical-characteristic-value obtaining means (44) that obtains an optical characteristic value in the region of interest (33) on the basis of the initial sound pressure and the corrected light intensity.

2.  The subject information obtaining apparatus according to Claim 1,
    wherein each of the weighting coefficients is based on an angle between a straight line that passes through the region of interest (33) and a detection surface of the corresponding acoustic wave detection element and a normal of the detection surface of the corresponding acoustic wave detection element.

3.  The subject information obtaining apparatus according to Claim 1 or 2,
    wherein each of the weighting coefficients is based on a distance from the region of interest (33) to the corresponding acoustic wave detection element.

4.  The subject information obtaining apparatus according to any one of Claims 1 to 3,
    wherein the acoustic wave detection elements (e1, e2, e3) include a first acoustic wave detection element and a second acoustic wave detection element,
    wherein the first acoustic wave detection element outputs a first detection signal by detecting the photoacoustic wave,
    wherein the second acoustic wave detection element outputs a second detection signal by detecting the photoacoustic wave,
    wherein the initial-sound-pressure obtaining means (42) obtains the initial sound pressure on the basis of the first detection signal and the second detection signal, and
    wherein the light-intensity obtaining means (43) obtains the corrected light intensity on the basis of a first weighting coefficient based on a sensitivity distribution of the first acoustic wave detection element, a second weighting coefficient based on a sensitivity distribution of the second acoustic wave detection element, and the light intensity of the light with which the region of interest (33) is irradiated.

5.  The subject information obtaining apparatus according to Claim 4,
    wherein the light-intensity obtaining means (43) obtains a first corrected light intensity in the region of interest (33) on the basis of the first weighting coefficient and the light intensity of the light, and a second corrected light intensity in the region of interest (33) on the basis of the second weighting coefficient and the light intensity of the light, and
    wherein the light-intensity obtaining means (43) obtains the corrected light intensity based on which the optical characteristic value is obtained on the basis of the first corrected light intensity and the second corrected light intensity.

6.  The subject information obtaining apparatus according to any one of Claims 1 to 3,
    wherein the acoustic wave detection elements (e1, e2, e3) output a third detection signal by detecting a first photoacoustic wave generated when the subject (30) is irradiated with first light and output a fourth detection signal by detecting a second photoacoustic wave generated when the subject (30) is irradiated with second light that is emitted at a time different from a time at which the first light is emitted,
    wherein the initial-sound-pressure obtaining means (42) obtains the initial sound pressure on the basis of the third detection signal and the fourth detection signal, and
    wherein the light-intensity obtaining means (43) obtains the corrected light intensity on the basis of the weighting coefficients based on the sensitivity distributions of the acoustic wave detection elements (e1, e2, e3), a light intensity of the first light with which the region of interest (33) is irradiated, and a light intensity of the second light with which the region of interest (33) is irradiated.

**7.** The subject information obtaining apparatus according to Claim 6,
wherein the light-intensity obtaining means (43) obtains a first corrected light intensity in the region of interest (33) on the basis of the light intensity of the first light and the weighting coefficients based on the sensitivity distributions of the acoustic wave detection elements (e1, e2, e3), and a second corrected light intensity in the region of interest (33) on the basis of the light intensity of the second light and the weighting coefficients based on the sensitivity distributions of the acoustic wave detection elements (e1, e2, e3), and
wherein the light-intensity obtaining means (43) obtains the corrected light intensity based on which the optical characteristic value is obtained on the basis of the first corrected light intensity and the second corrected light intensity.

**8.** A subject information obtaining method for obtaining an optical characteristic value on the basis of detection signals output from a plurality of acoustic wave detection elements (e1, e2, e3), each of which detects a photoacoustic wave generated when a subject (30) is irradiated with light, the subject information obtaining method comprising:

obtaining an initial sound pressure in a region of interest (33) in the subject (30) on the basis of the detection signals;
obtaining a corrected light intensity in the region of interest (33) on the basis of weighting coefficients based on sensitivity distributions of the acoustic wave detection elements (e1, e2, e3) and a light intensity of the light with which the region of interest (33) is irradiated; and
obtaining an optical characteristic value in the region of interest (33) on the basis of the initial sound pressure and the corrected light intensity.

**9.** The subject information obtaining method according to Claim 8,
wherein each of the weighting coefficients is based on an angle between a straight line that passes through the region of interest (33) and a detection surface of the corresponding acoustic wave detection element and a normal of the detection surface of the corresponding acoustic wave detection element.

**10.** The subject information obtaining method according to Claim 8 or 9,
wherein each of the weighting coefficients is based on a distance from the region of interest (33) to the corresponding acoustic wave detection element.

**11.** A program for causing a computer to execute the subject information obtaining method according to any one of Claims 8 to 10.

# FIG. 1

# FIG. 2

| | |
|---|---|
| DETECT PHOTOACOUSTIC WAVE GENERATED WHEN SUBJECT IS IRRADIATED WITH LIGHT | S100 |
| OBTAIN INITIAL SOUND PRESSURE IN REGION OF INTEREST BY USING DETECTION SIGNALS | S200 |
| DETERMINE WEIGHTING COEFFICIENTS ON THE BASIS OF SENSITIVITY DISTRIBUTIONS OF ACOUSTIC WAVE DETECTION ELEMENTS | S300 |
| OBTAIN CORRECTED LIGHT INTENSITY IN REGION OF INTEREST ON THE BASIS OF WEIGHTING COEFFICIENTS AND LIGHT INTENSITY OF LIGHT WITH WHICH REGION OF INTEREST IS IRRADIATED | S400 |
| OBTAIN OPTICAL CHARACTERISTIC VALUE IN REGION OF INTEREST USING INITIAL SOUND PRESSURE AND CORRECTED LIGHT INTENSITY IN REGION OF INTEREST | S500 |

# FIG. 3

# FIG. 4

Europäisches
Patentamt

European
Patent Office

Office européen
des brevets

# EUROPEAN SEARCH REPORT

Application Number

EP 13 15 7941

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2011/125468 A1 (CANON KK [JP]; MIYASATO TAKURO [JP]) 13 October 2011 (2011-10-13) | 1,6,8,11 | INV. A61B5/00 |
| Y | * paragraphs [0005], [0037] - [0049], [0060] * * figures 1,2,5 * * claim 9 * | 2-5,7,9, 10 | |
| | ----- | | |
| X | WO 2011/070775 A1 (CANON KK [JP]; SUDO YOSHIAKI [JP]; SATO AKIRA [JP]) 16 June 2011 (2011-06-16) | 1,8,11 | |
| Y | * paragraphs [0018], [0050] * * figures 4,5 * | 7 | |
| | ----- | | |
| X,D | US 2010/087733 A1 (NAKAJIMA TAKAO [JP] ET AL) 8 April 2010 (2010-04-08) | 1,8 | |
| A | * figures 3,4 * | 2-7,9-11 | |
| | ----- | | |
| Y | US 2011/251475 A1 (TOKITA TOSHINOBU [JP] ET AL) 13 October 2011 (2011-10-13) * paragraphs [0044], [0046] * * figures 4,5 * | 2-5,9,10 | |
| | ----- | | TECHNICAL FIELDS SEARCHED (IPC) |
| | | | A61B |

The present search report has been drawn up for all claims

| Place of search | Date of completion of the search | Examiner |
|---|---|---|
| The Hague | 11 July 2013 | Worms, Georg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 13 15 7941

This annex lists the patent family members relating to the patent documents cited in the above-mentioned European search report.
The members are as contained in the European Patent Office EDP file on
The European Patent Office is in no way liable for these particulars which are merely given for the purpose of information.

11-07-2013

| Patent document cited in search report | | Publication date | Patent family member(s) | | Publication date |
|---|---|---|---|---|---|
| WO 2011125468 | A1 | 13-10-2011 | CN<br>EP<br>JP<br>US<br>WO | 102821677 A<br>2555669 A1<br>2011217914 A<br>2013006089 A1<br>2011125468 A1 | 12-12-2012<br>13-02-2013<br>04-11-2011<br>03-01-2013<br>13-10-2011 |
| WO 2011070775 | A1 | 16-06-2011 | JP<br>US<br>WO | 2011120796 A<br>2012285248 A1<br>2011070775 A1 | 23-06-2011<br>15-11-2012<br>16-06-2011 |
| US 2010087733 | A1 | 08-04-2010 | JP<br>US | 2010088627 A<br>2010087733 A1 | 22-04-2010<br>08-04-2010 |
| US 2011251475 | A1 | 13-10-2011 | CN<br>EP<br>JP<br>US<br>WO | 102256537 A<br>2381837 A1<br>2010167258 A<br>2011251475 A1<br>2010074104 A1 | 23-11-2011<br>02-11-2011<br>05-08-2010<br>13-10-2011<br>01-07-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• JP 2010088627 A